# EUROPEAN PATENT SPECIFICATION

(11) **EP 0 919 218 B1**
(45) Date of publication and mention of the grant of the patent: **19.05.2004**
(21) Application number: 98907242.6
(22) Date of filing: 16.03.1998
(51) Int. Cl.: A61K 7/00, A61K 7/48, A61K 35/78

(54) **DERMATOLOGICAL PREPARATIONS FOR WHITENING THE SKIN**
DERMATOLOGISCHE ZUSAMMENSETZUNGEN ZUR BLEICHUNG DER HAUT
PREPARATIONS DERMATOLOGIQUES POUR BLANCHIR LA PEAU

(30) Priority: 19.03.1997 JP 8574697
(43) Date of publication of application: 02.06.1999
(73) Proprietor: SHISEIDO COMPANY LIMITED, Chuo-ku, Tokyo 104-8010 (JP)
(72) Inventor: YAGI, Eiichiro, Shiseido Res. Cnt Shiseido co. Ltd, Kanagawa 223-8553 (JP); SUZUKI, Rikako, Shiseido Res. Cnt Shiseido co. Ltd, Kanagawa 223-8553 (JP); NAGANUMA, Masako, Shiseido Res Cnt Shiseido Co Ltd, Kanagawa 223-8553 (JP); OHTA, Masahiro, Shiseido Res Cnt Shiseido co. Ltd., Kanagawa 223-8553 (JP)
(74) Representative: TER MEER STEINMEISTER & PARTNER GbR
(86) International application number: PCT/JP1998/001095
(87) International publication number: WO 1998/041183

(56) References cited:
- EP-A- 0 792 646
- FR-A- 2 669 032
- JP-A- 5 345 705
- JP-A- 8 012 565
- JP-A- 9 249 578
- DATABASE WPI Week 9330 Derwent Publications Ltd., London, GB; AN 1993-239913 XP002131333 & JP 05 163135 A (SUNTORY)
- PATENT ABSTRACTS OF JAPAN vol. 1996, no. 07 (C & JP 08 067616 A (NANBA TSUNEO ET AL.)
- PATENT ABSTRACTS OF JAPAN vol. 1998, no. 06 (C & JP 10 036216 A (POLA CHEM IND), 10 February 1998 (1998-02-10)
- PATENT ABSTRACTS OF JAPAN vol. 1999, no. 12 (C & JP 11 189514 A (LION CORP), 13 July 1999 (1999-07-13)
- PATENT ABSTRACTS OF JAPAN vol. 1999, no. 14 (C, 22 December 1999 (1999-12-22) & JP 11 246344 A (SHISEIDO CO)
- PATENT ABSTRACTS OF JAPAN vol. 1999, no. 14 (C, 22 December 1999 (1999-12-22) & JP 11 246343 A (SHISEIDO CO)
- STN, File Supplier, Karlsruhe, DE, File Chemical Abstracts, Vol 126, AN=122348 * abstract * XP002137737

## Description

### FIELD OF THE INVENTION

This invention relates to a whitening endermic liniment which, because it contains an extract from a plant of the Solanaceae family, genus Solanum, suppresses production of melanin and is effective in the prevention of and improvement in pigment deposition, chloasma, freckles, liver spots, etc. after sun exposure.

### BACKGROUND OF THE INVENTION

The mechanism of the development of chloasma and such on skin, although there are some unknown details, is generally believed to result from the formation of melanin pigment due to hormonal abnormalities or ultraviolet light stimulation from sunlight followed by abnormal deposition of this pigment in the skin.

This melanin pigment which causes the coloring of the skin is produced in melanin producing granules (melanosomes) in melanin cells (melanocytes) between the epidermis and the corium. Melanin thus produced is then diffused to neighboring cells by means of osmosis. The biochemical reactions in the melanocytes are speculated to be those described below.

That is, the production process of melanin pigment is thought to be as follows: tyrosine, one of the essential amino acids, becomes dopaquinone through the action of the enzyme tyrosinase, and this is then changed to a red pigment, to a colorless pigment and finally to melanin, which is black, by enzymatic as well as non-enzymatic oxidation.

Therefore, in order to suppress the production of melanin, it is important to suppress the first stage of the reactions, i.e. the action of tyrosinase.

However, compounds which suppress tyrosinase action, with the exception of hydroquinone, work very slowly and do not give sufficient improvement in pigment deposition in the skin.

On the other hand, hydroquinone, although its effects are recognized, has the problem of sensitization and therefore its uses are generally limited. For the purpose of improving its safety, attempts have been made to modify it into a monoester of a higher fatty acid, an alkyl monoether and such (Japanese unexamined patent publication Tokkai Sho 58-154507). However, esters are decomposed by hydrolytic enzymes in the body and therefore are not necessarily safe. Sufficiently safe ethers have not been obtained yet either.

For the purpose of solving these problems, the inventors investigated a wide variety of substances for a melanin production suppression effect, and discovered that an extract from a plant of the Solanaceae family, genus Solanum, had melanin production suppression and tyrosinase inhibition actions and thus completed the present invention.

An extract of a plant of the Solanaceae family, genus Solanum, was reported to have been used in an endermic liniment for improvement in the skin with pimples (Japanese unexamined patent publication Tokkai Sho 56-154500). However, applications of this extract in melanin production suppression actions or in whitening agents is not known at all. The inventors completed the present invention based on this finding.

The object of the present invention is to provide a whitening endermic liniment which has superior melanin production suppression effect and tyrosinase inhibition effect and is superior in terms of safety.

### DISCLOSURE OF THE INVENTION

That is, the present invention is a whitening endermic liniment which characteristically contains an extract from a plant of the Solanaceae family, genus Solanum, wherein the plant is jurubeba paiz (Solanum paniculatum) wherein the blend ratio of the extract from the plant of the Solanaceae family, genus Solanum, is 0.0005 - 10.0 wt%.

### THE BEST MODES OF THE EMBODIMENTS

The present invention is described in detail below.

The plant of the Solanaceae family, genus Solanum, used in the present invention, jurubeba paiz (scientific name: Solanum paniculatum) is found on dry grassy plains and pastures particularly in Brazil.

The melanin production suppression effect and the tyrosinase inhibition effect of the extract of the plant of the Solanaceae family, genus Solanum, was discovered by the inventor for the first time. Its application to whitening agents and whitening endermic liniments is not known at all.

The extract used in the present invention is obtained by immersing or heated refluxing of roots, leaves, tubers, stems, fruits, etc. or the whole aforementioned plant in an extraction solvent, followed by filtering and condensation. The extraction solvent used in the present invention can be any solvent which is normally used for extraction. Examples include alcohols such as methanol and ethanol, hydrated alcohols, and organic solvents such as acetone and ethyl acetate, and these can be used either independently or in combination.

In the present invention, the blend ratio of the extract from the aforementioned plants, in a dry form, is 0.0005 - 10.0 wt%, preferably 0.01 - 5.0 wt%, of the total endermic liniment. If it is less than 0.0005 wt% then the effects of the present invention cannot be sufficiently achieved, and if it is more than 10 wt% then pharmaceutical preparation becomes difficult. Therefore neither case is preferable. No significant increase in the effect is observed when using more than 5 wt%.

In addition to the essential ingredient described above, the endermic liniment of the present invention can contain, as necessary, those ingredients such as are normally used in cosmetics, drugs, etc. in the form of an endermic liniment, including other whitening agents, humectants, antioxidants, oil-based ingredients, ultraviolet light absorbents, surfactants, thickeners, alcohols, powder ingredients, colorings, water-based ingredients, water and various skin nutrients.

In addition, sequestering agents including disodium edetate, trisodium edetate, sodium citrate, sodium polyphosphate, sodium metaphosphate and gluconic acid, drugs including caffeine, tannin, verapamil, tranexamic acid and its derivatives, glycyrrhiza extract, glabridin, various crude drugs, tocopherol acetate, glycyrrhizic acid and its derivatives or its salts, whitening agents including vitamin C, ascorbic acid phosphate magnesium, ascorbyl glucoside, arbutin and kojic acid, and sugars including glucose, fructose, mannose, sucrose and trehalose can also be added.

The endermic liniment of the present invention can be in any form which is conventionally used as an endermic liniment, including ointment, cream, emulsion, lotion, facial packs and bath additives.

### EXAMPLES

The present invention is described in detail below by referring to examples. The present invention is not limited to these examples. The blend ratios are in weight percent units. Before explaining the examples, the testing methods and the results of the melanin suppression effect, tyrosinase activity inhibition effect and whitening effect of the plant extract of the present invention are described.

### The testing methods and results

### 1. Sample preparation

100 g of the root part of jurubeba paiz was immersed in ethanol at room temperature for a week. The extract solution was then concentrated to obtain 1.6 g of an ethanol extract. This extract was dissolved in DMSO to obtain a 1% solution, and this solution was diluted to adjust the concentration for the following experiments.

### 2. Cell culture

B16 melanoma culture cells from mice were used. A culture was conducted in a CO₂ incubator (95% air and 5% carbon oxide) at 37°C using Eagle's medium containing 10% FBS and theophylline (0.09 mg/ml). After 24 hours of culturing, the sample solution was added to it such that the final concentration (in dried extract) was 10⁻² - 10⁻⁵ wt% for jurubeba paiz. The culture was continued for 3 more days, following which time melanin production was visually evaluated and the tyrosinase activity inihibition effect was measured.

### 3. Visual evaluation of the amount of melanin

A diffusion plate was placed on top of the lid of the well plate, and the amount of melanin in the cells was evaluated using an inverted microscope. The evaluation was compared with that of a sample with no added extract from the plant of the Solanaceae family (control sample). The results are shown in Table 1.

For a reference, the same testing was conducted on Nepeta japonica ( Lamium album subfamily, perilla family) extract which was already known to suppress melanin production. These results are also shown in Table 1.

### <Criteria>

○ : Whiter than the control (the amount of melanin is less than that in the control)
Δ : Somewhat whiter (the amount of melanin is somewhat less than that in the control)
×: Comparable degree of whiteness as the control.

### 4. Tyrosinase activity measurement

Before the measurement, the medium in the well(s) was removed, followed by washing twice with 100 microliters of PBS. 45 microliters of PBS containing 1% Triton X (surfactant from Rohm & Haas) was then added to each well. The plate was vibrated for 1 minute to thoroughly destroy the cell membranes, and the absorbance at 475 nm was measured using a microplate reader, which was defined as the absorbance at time 0 minutes. Quickly after this, 5 microliters of 10 mM L-DOPA solution was added and the plate was transferred to an incubator kept at 37°C to react for 60 minutes. The plate was vibrated for 1 minute and the absorbance (475 nm) at time 60 minutes was measured-The tyrosinase activity ratio (%) was defined as a ratio of the absorbance difference between time 0 minutes and time 60 minutes for the sample to which the plant extract was added and the absorbance difference between time 0 minutes and time 60 minutes for the sample to which the plant extract was not added. The results are shown in Table 1.

For a reference, the same testing was conducted on Nepeta japonica extract which was already known to inhibit tyrosinase activity. These results are also shown in Table 1. In the table, "-" indicates that no significant difference compared with the control was observed within a 5% level of significance. Empty columns indicate that testing was not conducted for those because the tyrosinase activity inhibition effect had been observed with a lower concentration.

**Table 1**

| Test | Visual evaluation of melanin production | | | | Tyrosinase activity ratio (%) | | | |
|---|---|---|---|---|---|---|---|---|
| Concentration (wt%) | 10⁻⁵ | 10⁻⁴ | 10⁻³ | 10⁻² | 10⁻⁵ | 10⁻⁴ | 10⁻³ | 10⁻² |
| Jurubeba paiz extract | × | **○** | | | 90 | 106 | | |
| Nepeta japonica extract | × | × | × | × | - | - | - | 55 |

### 5. Whitening effect testing

### [Test method]

40 testees were exposed to artificial light (UV-A + UV-B) for 30 minutes (10 minutes a day for 3 days) and the skin of an inner lateral part of their upper arm was used as the subject of the test. Beginning after 5 days from the day they were exposed to the sunlight, each sample was applied to this skin once in the morning and once in the afternoon for 4 weeks. The panel was divided into 5 groups with 8 persons in each group. Testing was conducted using the following formulations.

| (Alcohol phase) | |
|---|---|
| 95% ethyl alcohol | 55.0 wt% |
| Polyoxyethylene (25-mole) hardened castor oil ether | 2.0 |
| Antioxidant/preservative | Appropriate amount |
| Perfume | Appropriate amount |
| Drug (specified in Table 2) | |

| (Water Phase) | |
|---|---|
| Glycerine | 5.0 |
| Sodium hexamethaphosphate | Appropriate amount |
| Ion exchange water | Balance |

### <Preparation method>

The water phase and the alcohol phase were prepared separately and then mixed and solubilized.

### [Evaluation method]

The hypochromic effect after the application was evaluated based on the criteria below.

### <Criteria>

ⓞ : Very effective or effective on 80% or more of the testees
○ : Very effective or effective on 50% to less than 80% of the testees
Δ: Very effective or effective on 30% to less than 50% testees
×: Very effective or effective on less than 30% of the testees

Samples were prepared with the blend compositions described in the aforementioned test method, and the drugs listed in Table 2 were used to compare the whitening effect. The results are shown in Table 2.

**Table 2**

| Drug | Blend ratio (wt%) | Effect |
|---|---|---|
| Nothing added | - | × |
| Hydroquinone | 1.0 | Δ |
| Jurubeba paiz extract | 0.1 | ○ |
| Jurubeba paiz extract | 1.0 | ○ |
| Jurubeba paiz extract | 5.0 | ⓞ |

The jurubeba paiz extracts in Table 2 were obtained by heated reduction of the root of jurubeba paiz in ethanol, followed by filtering and concentration/drying.

As clearly shown in Table 2, it was confirmed that the samples with Jurubeba paiz extract, more effectively prevented excessive deposition of the melanin pigment and thus prevented darkening of the skin.

Examples of the whitening endermic liniment of the present invention are shown below.

In "the whitening endermic liniment containing an extract from a plant of the Solanaceae family, genus Solanum", the jurubeba paiz extract was obtained by heated reduction of the root of jurubeba paiz in each extraction solvent, followed by filtering and concentration/drying.

(1) "The whitening endermic liniment containing an extract from a plant of the Solanaceae family, genus Solanum; jurubeba paiz (Solanum paniculatum)".

### Example 1 Cream

| (Recipe) | |
|---|---|
| Stearic acid | 5.0 wt% |
| Stearyl alcohol | 4.0 |
| Isopropyl myristate | 18.0 |
| Glycerine monostearic ester | 3.0 |
| Propylene glycol | 10.0 |
| Jurubeba paiz methanol extract | 0.01 |
| Caustic potash | 0.2 |
| Preservative | Appropriate amount |
| Perfume | Appropriate amount |
| Ion exchange water | Balance |

### (Preparation method)

Propylene glycol, the jurubeba paiz extract and caustic potash were added to the ion exchange water and the mixture was heated to and kept at 70°C (water phase). The other ingredients were mixed, then heat-melted and the temperature was kept at 70°C (oil phase). The oil phase was gradually added to the water phase, and after all of it had been added the temperature was kept at that temperature to allow the mixture to react. Finally, the mixture was homogeneously emulsified by a homogenizer and cooled to 30°C while being thoroughly stirred.

### Example 2 Cream

| (Recipe) | |
|---|---|
| Stearic acid | 2.0 wt% |
| Stearyl alcohol | 7.0 |
| Hydrated lanolin | 2.0 |
| Squalane | 5.0 |
| 2-octyldodecyl alcohol | 6.0 |
| Polyoxyethylene (25-mole) cetyl alcohol ether | 3.0 |
| Glycerine monostearic ester | 2.0 |
| Propylene glycol | 5.0 |
| Jurubeba paiz ethanol extract | 0.05 |
| Ethyl paraben | 0.3 |
| Perfume | Appropriate amount |
| Ion exchange water | Balance |

### (Preparation method)

Propylene glycol was added to the ion exchange water and the mixture was heated to and kept at 70°C (water phase). The other ingredients were mixed, then heat-melted and the temperature was kept at 70°C (oil phase). The oil phase was added to the water phase, and after pre-emulsification, the mixture was homogeneously emulsified by a homogenizer and cooled to 30 °C while being thoroughly stirred.

### Example 3 Cream

| (Formula) | |
|---|---|
| Solid paraffin | 5.0 wt% |
| Bees wax | 10.0 |
| Vaseline | 15.0 |
| Liquid paraffin | 41.0 |
| Glycerine monostearic ester Polyoxyethylene (20-mole) | 2.0 |
| sorbitan monolauric ester | 2.0 |
| Soap powder | 0.1 |
| 2-ethylhexyl paramethoxycinnamate | 1.5 |
| Borax | 0.2 |
| Jurubeba paiz acetone extract | 0.05 |
| Jurubeba paiz ethanol extract | 0.05 |
| Ethyl paraben | 0.3 |
| Perfume | Appropriate amount |
| Ion exchange water | Balance |

### (Preparation method)

Soap powder and borax were added to the ion exchange water and the mixture was heated to and kept at 70°C (water phase). The other ingredients were mixed, then heat-melted and the temperature was kept at 70°C (oil phase). The oil phase was gradually added to the water phase while stirring was conducted to allow the reaction to occur. When the reaction was complete, the mixture was homogeneously emulsified by a homogenizer and then cooled to 30°C while being thoroughly stirred.

### Example 4 Essence

| (Recipe) | |
|---|---|
| (A phase) | |
| Ethyl alcohol (95%) | 10.0 wt% |
| Polyoxyethylene (20-mole) octyl dodecanol | 1.0 |
| Pantothenyl ethyl ether | 0.1 |
| Jurubeba paiz methanol extract | 1.5 |
| Ascorbyl glucoside | 1.5 |
| Arbutin | 3.0 |
| Methyl paraben | 0.15 |

| (B phase) | |
|---|---|
| Potassium hydroxide | 0.1 |

| (C phase) | |
|---|---|
| Glycerine | 5.0 |
| Dipropylene glycol | 10.0 |
| Sodium hydrogen sulfite | 0.03 |
| Carboxyvinyl polymer | 0.2 |
| (Product name: Carbopol 940 from B.F. Goodrich Chemical company) | |
| Purified water | Balance |

### (Preparation method)

The A phase and the C phase were independently dissolved homogeneously, and then the A phase was added to the C phase and solubilized. The B phase was then added, and finally containers were filled.

### Example 5 Facial pack

| (Recipe) | |
|---|---|
| (A phase) | |
| Dipropylene glycol | 5.0 wt% |
| Polyoxyethylene (60-mole) hardened castor oil | 5.0 |

| (B phase) | |
|---|---|
| Jurubeba paiz methanol extract | 0.01 |
| Olive oil | 5.0 |
| Tocopherol acetate | 0.2 |
| Ethyl paraben | 0.2 |
| Perfume | 0.2 |

| (C phase) | |
|---|---|
| Ascorbyl 2-glucoside | 2.0 |
| Polyvinyl alcohol | 13.0 |
| (Degree of saponification 90, degree of polymerization 2,000) Ethanol | 7.0 |
| Purified water | Balance |

### (Preparation method)

The A, B and C phases were independently dissolved homogeneously, and then the B phase was added to the A phase and solubilized. The C phase was then added to this, and finally containers were filled.

### Example 6 Solid foundation

| (Recipe) | |
|---|---|
| Talc | 43.1 wt% |
| Kaolin | 15.0 |
| Sericite | 10.0 |
| Zinc flower | 7.0 |
| Titanium dioxide | 3.8 |
| Yellow iron oxide | 2.9 |
| Black iron oxide | 0.2 |
| Squalane | 8.0 |
| Isostearic acid | 4.0 |
| POE sorbitan monooleate | 3.0 |
| Isocetyl octate | 2.0 |
| Jurubeba paiz ethanol extract | 1.0 |
| Preservative | Appropriate amount |
| Perfume | Appropriate amount |

### (Preparation method)

The powder ingredients, i.e. from talc to black iron oxide, were thoroughly mixed by a blender, and the oil-based ingredients, i.e. from squalene to isocetyl octate, and Jurubeba paiz ethanol extract, the preservative and the perfume were added to this. After a thorough kneading, the product was poured into containers and molded.

### Example 7 Emulsified foundation (cream type)

| (Recipe) | |
|---|---|
| (Powder portion) | |
| Titanium dioxide | 10.3 wt% |
| Sericite | 5.4 |
| Kaolin | 3.0 |
| Yellow iron oxide | 0.8 |
| Red iron oxide | 0.3 |
| Black iron oxide | 0.2 |

| (Oil phase) | |
|---|---|
| Decamethylpentasiloxane | 11.5 |
| Liquid paraffin | 4.5 |
| Polyoxyethylene modified dimethylpolysiloxane | 4.0 |

| (Water Phase) | |
|---|---|
| Purified water | 50.0 |
| 1,3-butylene glycol | 4.5 |
| Jurubeba paiz ethanol extract | 1.5 |
| Sorbitan sesquioleic ester | 3.0 |
| Preservative | Appropriate amount |
| Perfume | Appropriate amount |

### (Preparation method)

After heating and stirring the water phase, the powder portion, thoroughly mixed and crushed, was added to it and the mixture was treated with a homogenizer. The heat-mixed oil phase was then added to this mixture and the resulting mixture was treated with a homogenizer. Finally, the perfume was added while the mixture was stirred and the temperature was lowered to room temperature.

### INDUSTRIAL APPLICABILITY OF THE INVENTION

As described thus far, the whitening endermic liniment of the present invention has a melanin production suppression action and a tyrosinase activity suppression action and therefore exhibits superior hypochromic effects and whitening effects on pigment deposition, chloasma, freckles, liver spots, etc. after sun exposure. This endermic liniment is also superior in terms of safety.

## Claims

1. A whitening endermic liniment which characteristically contains an extract from a plant of the Solanaceae family, genus Solanum,
wherein the plant of the Solanaceae family, genus Solanum, is jurubeba paiz (scientific name: Solanum paniculatum), and
wherein the blend ratio of the extract from the plant of the Solanaceae family, genus Solanum, is 0.0005 - 10.0 wt%.

## Patentansprüche

1. Bleichende endermatische Salbe, welche charakterischerweise einen Extrakt einer Pflanze der Familie Solanaceae, Gattung Solanum, enthält,
wobei die Pflanze der Familie Solanaceae, Gattung Solanum, jurubeba paiz (wissenschaftliche Bezeichnung: Solanum paniculatum) ist, und
wobei das Mischungsverhältnis des Extrakts aus der Pflanze der Familie Solanaceae, Gattung Solanum, 0,0005-10,0 Gew.-% beträgt.

## Revendications

1. Liniment intradermique blanchissant qui contient, de manière caractéristique, un extrait d'une plante de la famille des solanacées, du genre *Solanum*,
dans lequel la plante de la famille des solanacées, du genre *Solanum*, est jurubeba (non scientifique : *Solanum paniculatum)*, et
dans lequel le rapport de mélange de l'extrait de la plante de la famille des solanacées, du genre *Solanum,* est de 0,0005 à 10,0 % en poids.
